# EUROPEAN PATENT SPECIFICATION

(11) **EP 2 055 332 B1**
(45) Date of publication and mention of the grant of the patent: **05.09.2012**
(21) Application number: 08253504.8
(22) Date of filing: 28.10.2008
(51) Int. Cl.: A61M 5/145

(54) **Drug delivery system with cartridge interlock**
Wirkstofffreisetzungssystem mit Kartuschensperre
Système d'administration de médicaments doté d'un verrouillage de cartouche

(30) Priority: 29.10.2007 US 983349 P
(43) Date of publication of application: 06.05.2009
(73) Proprietor: Animas Corporation, West Chester, PA 19380 (US)
(72) Inventor: O'Connor, Sean, West Chester, PA 19380 (US)
(74) Representative: Tunstall, Christopher Stephen

(56) References cited:
- US-A- 5 947 935
- US-B1- 6 475 192
- US-B1- 6 569 127
- US-B1- 6 984 222

## Description

### FIELD OF THE INVENTION

The present invention relates, in general, to cartridges used in drug delivery devices and, more particularly, to cartridges with an interlock and methods for their use.

### BACKGROUND OF THE INVENTION

The use of drug delivery devices for various types of drug therapy is becoming more common as the automated infusion of a drug may provide more reliable and more precise treatment to a patient.

US5947935 describes a connection mechanism adapted to make a releasable connection between a syringe plunger and a drive member of an injector. The drive member controls the movement of the syringe plunger.

US6984222 describes a dynamic wedge seal which is said to improve the sealing engagement between the plunger cover and syringe barrel.

Diabetes is a major health concern, as it can significantly impede on the freedom of action and lifestyle of persons afflicted with this disease. Typically, treatment of the more severe form of the condition, Type I (insulin-dependent) diabetes, requires one or more insulin injections per day, referred to as multiple daily injections. Insulin is required to control glucose or sugar in the blood, thereby preventing hyperglycemia which, if left uncorrected, can lead to ketosis. Additionally, improper administration of insulin therapy can result in hypoglycemic episodes, which can cause coma and death. Hyperglycemia in diabetics has been correlated with several long-term effects of diabetes, such as heart disease, atherosclerosis, blindness, stroke, hypertension, and kidney failure.

The value of frequent monitoring of blood glucose as a means to avoid or at least minimize the complications of Type I diabetes is well established. Patients with Type II (non-insulin-dependent) diabetes can also benefit from blood glucose monitoring in the control of their condition by way of diet and exercise. Thus, careful monitoring of blood glucose levels and the ability to accurately and conveniently infuse insulin into the body in a timely manner is a critical component in diabetes care and treatment.

In order to more effectively control diabetes in a manner that reduces the limitations imposed by this disease on the lifestyle of the affected person, various devices for facilitating blood glucose (BG) monitoring have been introduced. Typically, such devices, or meters, permit the patient to quickly, and with a minimal amount of physical discomfort, obtain a sample of their blood or interstitial fluid which is then analyzed by the meter. In most cases, the meter has a display screen which shows the BG reading for the patient. The patient may then dose themselves with the appropriate amount, or bolus, of insulin. For many diabetics, this results in having to receive multiple daily injections of insulin. In many cases, these injections are self-administered.

Due to the debilitating effects that abnormal BG levels can have on patients, i.e., hyperglycemia, persons experiencing certain symptoms of diabetes may not be in a situation where they can safely and accurately self-administer a bolus of insulin. Moreover, persons with active lifestyles find it extremely inconvenient and imposing to have to use multiple daily injections of insulin to control their blood sugar levels, as this may interfere or prohibit their ability to engage in certain activities. For others with diabetes, multiple daily injections may simply not be the most effective means for controlling their BG levels. Thus, to further improve both accuracy and convenience for the patient, insulin infusion pumps have been developed.

Insulin pumps are generally worn on the patient's body, either above or below their clothing. These relatively small, unobtrusive devices typically store a quantity of insulin in a replaceable cartridge and include a processing unit, a display screen, and input functions such as buttons or a keypad. Such pumps may include the ability to run multiple insulin delivery programs, such as basal and bolus programs, to eliminate the need for injections of insulin via needles and syringes, by providing medication via an infusion device that can be worn by the patient for an extended period of time, usually in the range of 1-3 days.

While the convenience of an insulin pump has helped to improve the lifestyle of diabetics and has lessened the impact of their disease on their normal activity, advances in insulin pumps are still needed. Insulin pumps that use a motor-driven piston to deliver insulin from a cartridge may be affected by pressure differentials between the cartridge and infusion site. Pressure differentials result from height variations between the cartridge and the infusion site, referred to as head height, and by changes in atmospheric pressure as may occur in an airplane. When the pressure differential produces a force that exceeds the holding force between cartridge plunger and barrel, the cartridge plunger will advance, causing unwanted delivery of insulin.

Therefore, it would be desirable for patients and caregivers to have an insulin pump and cartridge system that includes a mechanism to prevent unwanted delivery of the drug.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

FIGS. 1A and 1B are perspective views of a cartridge and a portion of a drug infusion pump according to an exemplary embodiment of the present invention;

FIG. 2 is a perspective view of a drug infusion system according to an exemplary embodiment of the present invention;

FIGS. 3A and 3B are perspective views of a cartridge according to an exemplary embodiment of the present invention;

FIG. 4 is a perspective view of a piston according to an exemplary embodiment of the present invention;

FIG. 5 is a perspective view of the proximal end of the cartridge chamber in a drug infusion pump according to an exemplary embodiment of the present invention;

FIGS. 6A - 6C, 7A - 7D and 8A - 8D are schematic perspective and partial cross-sectional views of a sequence of steps in a process for priming a drug infusion pump in which the cartridge is completely filled with a drug;

FIGS. 9A - 9B and 10A - 10C are schematic perspective and partial cross-sectional views of a sequence of steps in a process for priming a drug infusion pump in which the cartridge is partially filled with a drug; and

FIGS. 11A -11D are schematic perspective views of a sequence of steps in a process for removing a cartridge from a drug infusion pump.

### DETAILED DESCRIPTION OF ILLUSTRATIVE EMBODIMENTS OF THE INVENTION

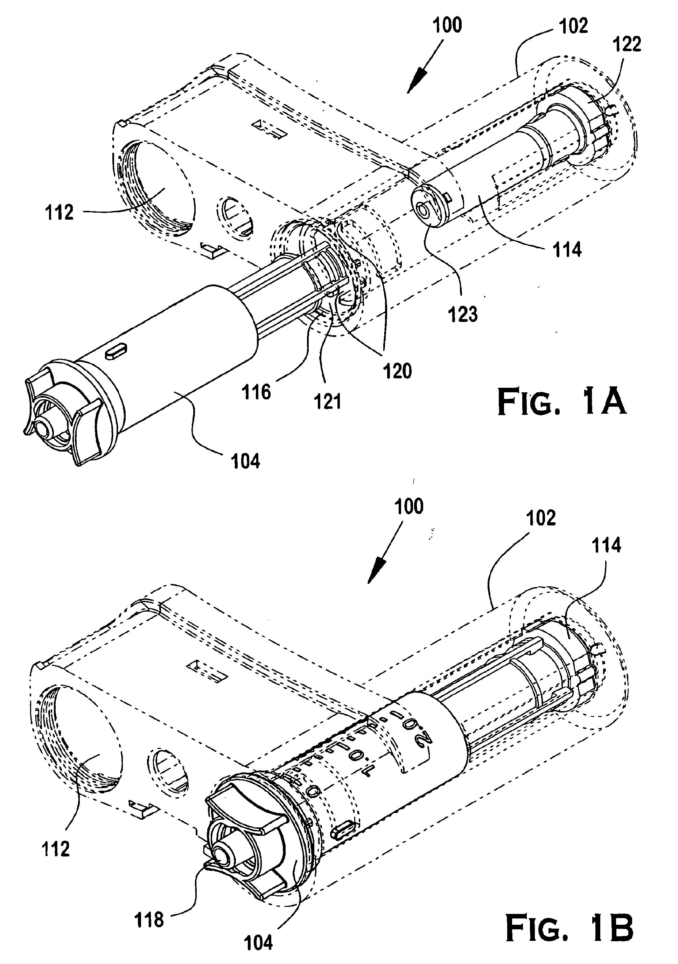

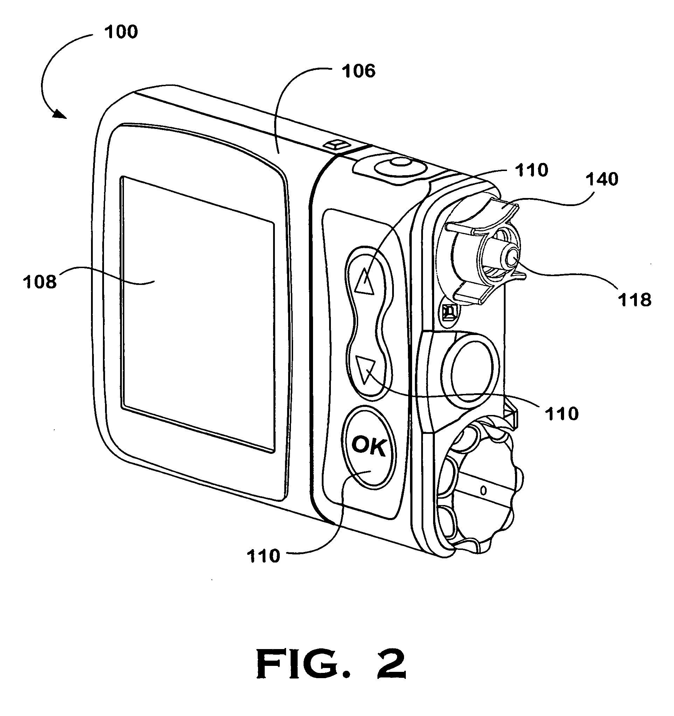

FIGS. 1A, 1B and 2 illustrate a drug delivery system 100 according to an exemplary embodiment of the present invention. The drug delivery system 100 includes a drug infusion pump 102 and a cartridge 104.

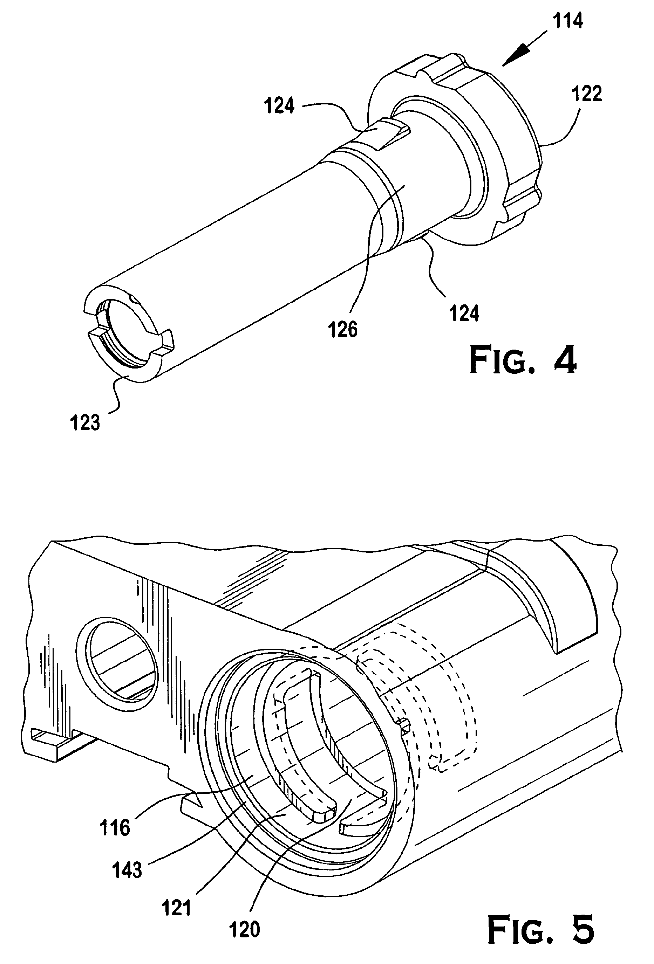

The drug infusion pump 102 includes a housing 106, a display 108 for providing operational information to the user, a plurality of navigational buttons 110 for the user to input information, a battery (not shown) in a battery compartment 112 for providing power to the drug infusion pump 102, processing electronics (not shown), a piston 114 and a motor (not shown) for forcing a drug from a cartridge 104 in a chamber 116 through a side port 118 connected to an infusion set (not shown) and into the body of the user. The chamber 116 includes at least one slot 120 on an inner surface 121 (see FIGS 1A and 5). The piston 114 includes a proximal end 122, a distal end 123, and at least one projection 124 on an outer surface 126 (see FIGS. 1A and 4).

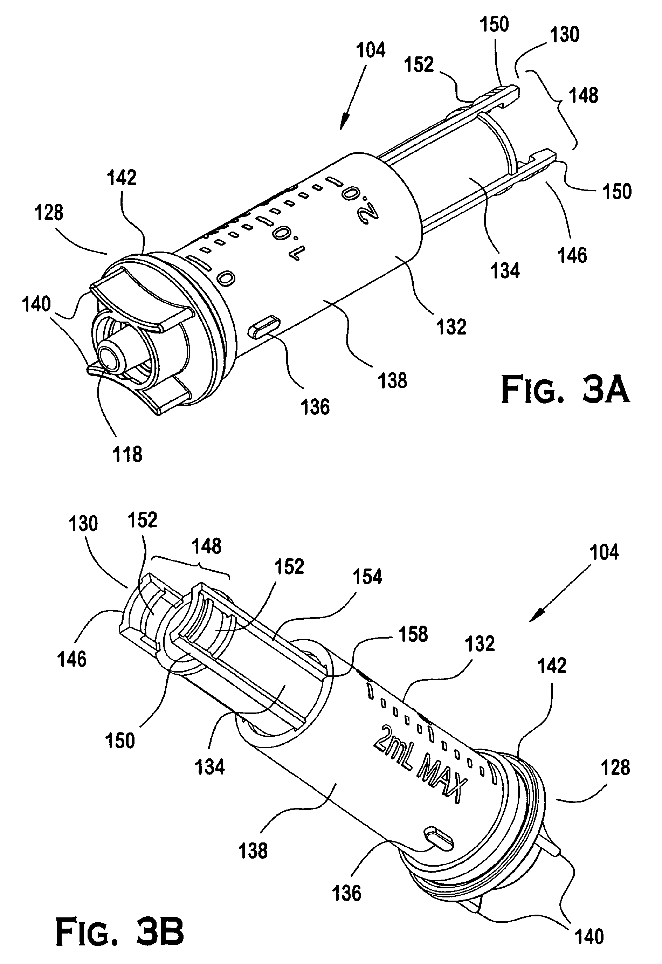

As illustrated in FIGS. 3A and 3B, the cartridge 104 is generally cylindrical in shape and includes a proximal end 128, a distal end 130, a hollow barrel 132 and a hollow plunger 134. The barrel 132 is configured to receive and store a drug and includes at least one tab 136 on an outer surface 138 that mates with and moves within the at least one slot 120 on the inner surface 121 of the chamber 116. The at least one tab 136 may be located on any portion of the outer surface 138 of the barrel 132 and the at least one slot 120 may be located on any portion of the inner surface 121 of the chamber 116 provided the at least one tab 136 can mate with and move within the at least one slot 120.

The cartridge 104 may also include a finger grip 140 on the proximal end 128 that facilitates insertion and removal of the cartridge 104. The finger grip 140 includes the side port 118 that is connected to the infusion set through which the drug is delivered to the body of the user. The finger grip 140 may be co-molded with the cartridge 104 during the manufacture of the cartridge 104, thus eliminating the need for a separate chamber cap to secure the cartridge 104 within the drug infusion pump 102.

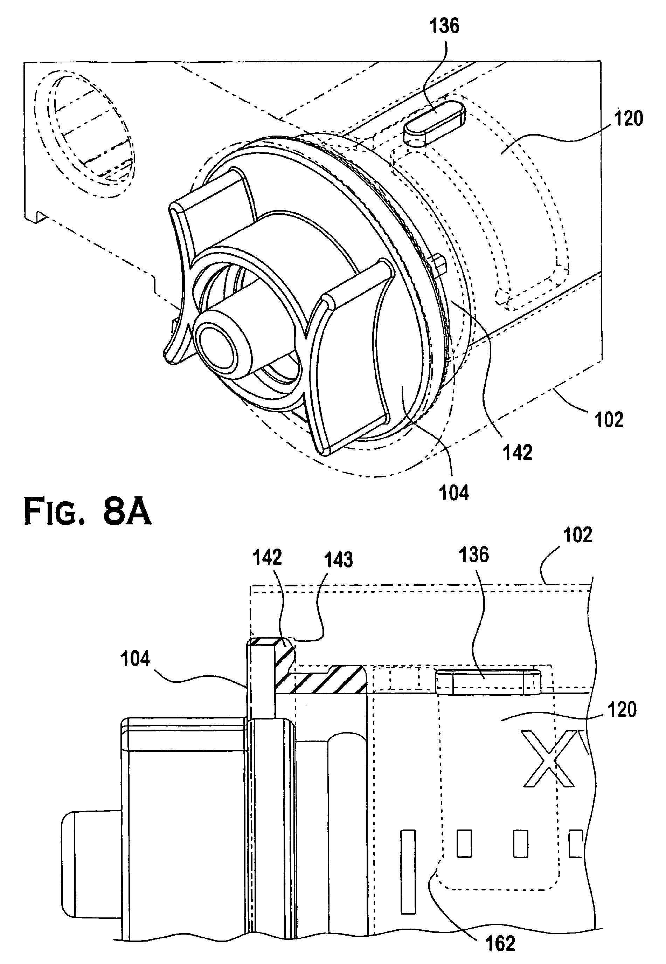

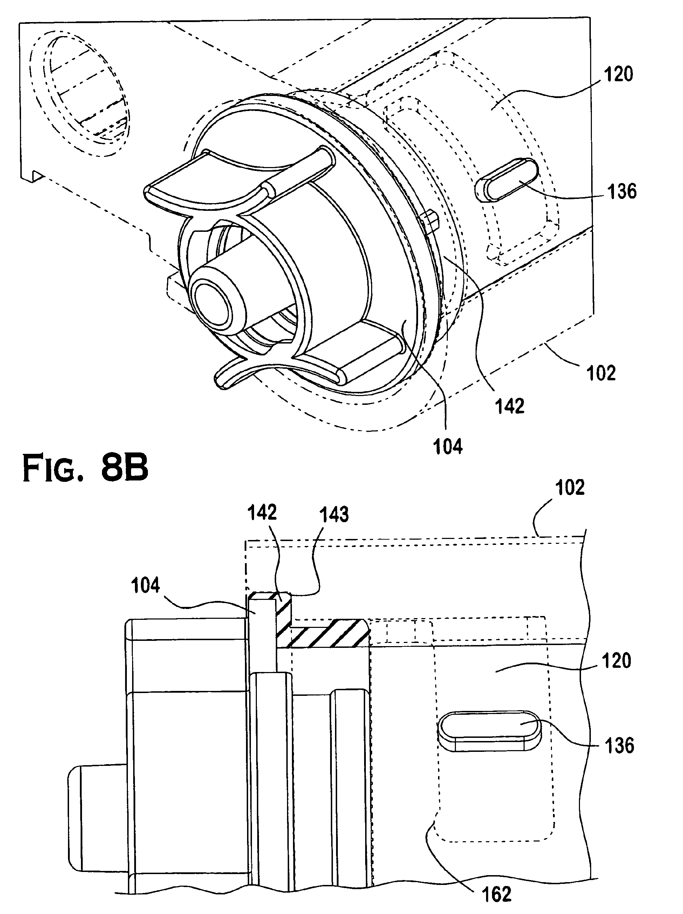

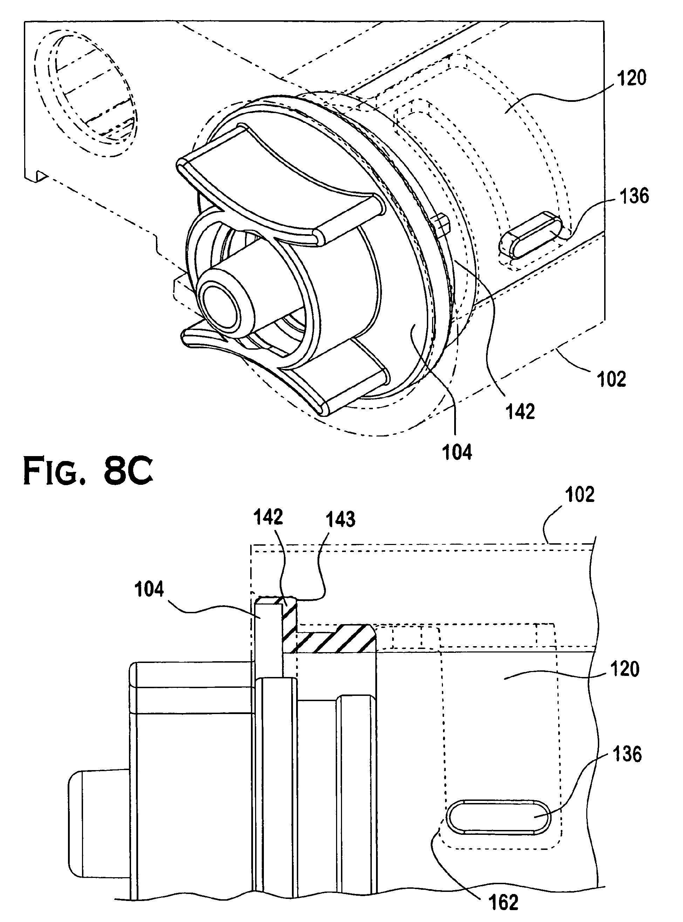

The cartridge 104 may further include an elastomeric spring retention feature 142 (e.g., an over-molded seal or gasket) that seals the cartridge 104 within the chamber 116 of the drug infusion pump 102. The spring retention feature 142 is compressed by a ledge 143 (shown in FIG. 5) when the cartridge 104 is inserted and rotated in the chamber 116 of the drug infusion pump 102, as will be described below with reference to FIGS. 8A - 8D. Spring retention feature 142 may be formed of deformable material such as rubber or thermoplastic elastomer.

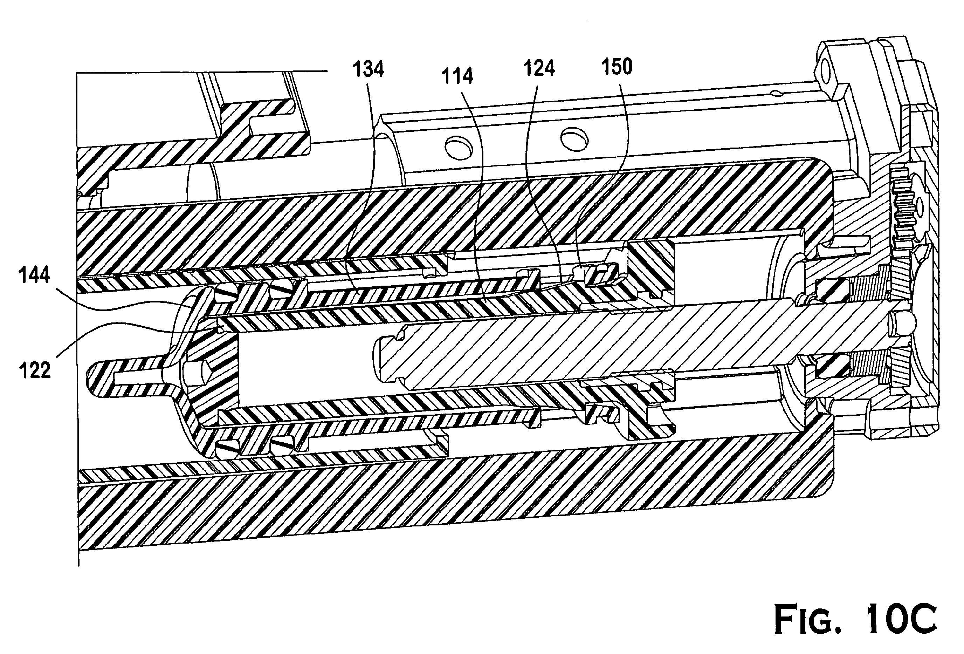

Referring again to FIGS. 3A and 3B, the plunger 134 includes a first end 144 (shown in FIG. 10C) and a second end 146. The first end of the plunger 134 is slidably inserted into the hollow barrel 132 of the cartridge 104 and the second end 146 of the plunger 134 is slidably inserted into the chamber 116 of the drug infusion pump 102 (see FIGS. 1A and 1B). As illustrated in FIGS. 3A and 3B, the second end 146 of the plunger 134 includes an interlock 148 that mates with the at least one projection 124 on the outer surface 126 of the piston 114. The interlock 148 may include at least one flexible arm 150 with an opening 152 for receiving the at least one projection 124 on the piston 114.

As illustrated in FIG. 3B, the plunger 134 also includes at least one keying feature 154 (e.g., a rail) on an outer surface that mates with at least one groove 158 on an inner surface of the barrel 132 to maintain the proper orientation of the opening in the arm 150 of the interlock 148 with the at least one tab 136 on the outer surface 138 of the barrel 132. The keying feature 154 may be oriented longitudinally on the outer surface of the plunger 134.

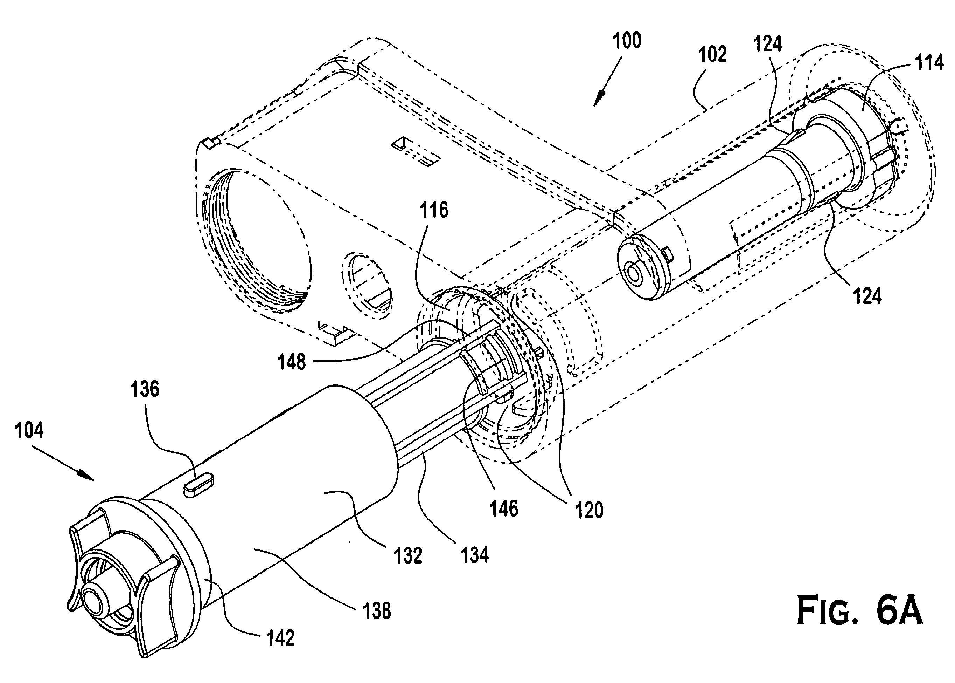

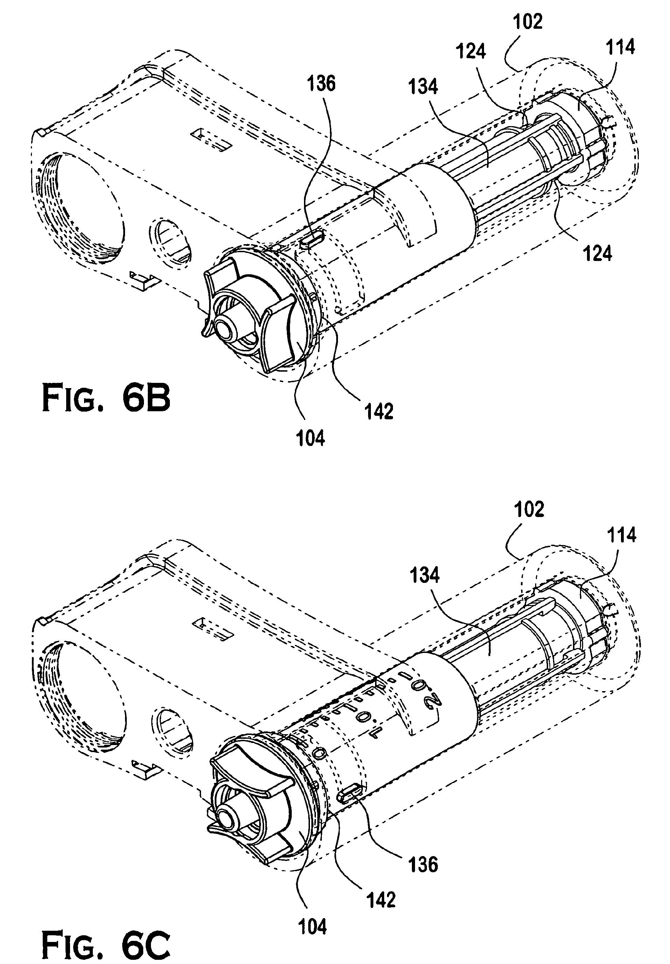

Referring to FIGS. 6A - 6C, 7A - 7D and 8A - 8D, a sequence of steps in a process for priming a pump 102 is illustrated in which the cartridge 104 is filled with a drug (e.g., insulin). The provision of an exemplary drug delivery system 100 that may be used with the subject method is depicted in FIGS. 1A, 1B and 2 in which like elements of the earlier figures are identified with like numerals.

In the first step of the subject method, a drug delivery system 100 is provided that includes a drug infusion pump 102 and a cartridge 104 (see FIG. 6A). The cartridge 104 includes a plunger 134 with an interlock 148 on a second end 146 according to exemplary embodiments of the present invention.

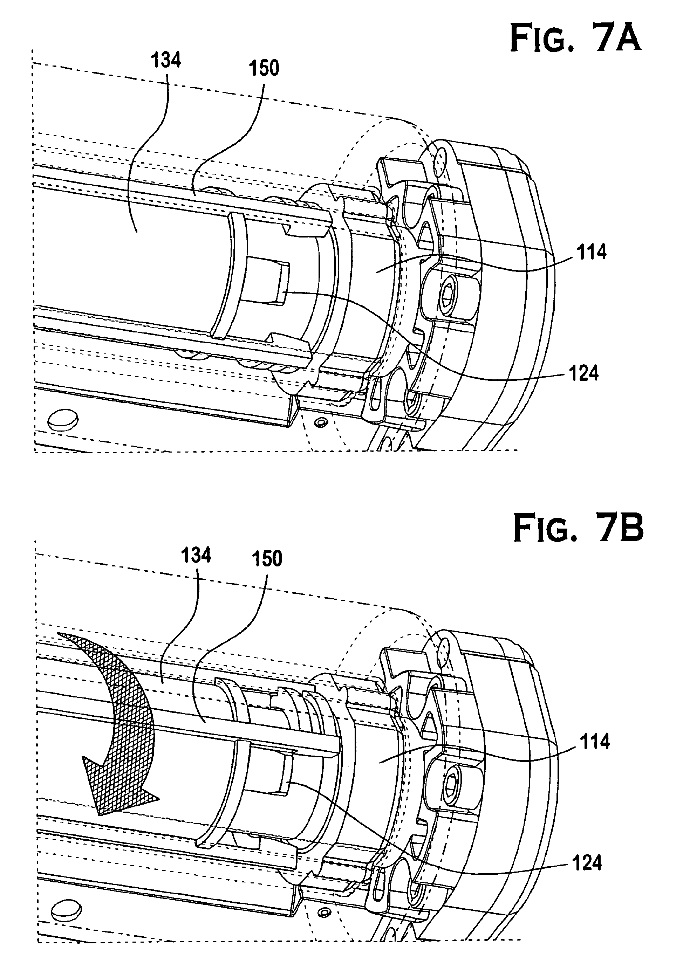

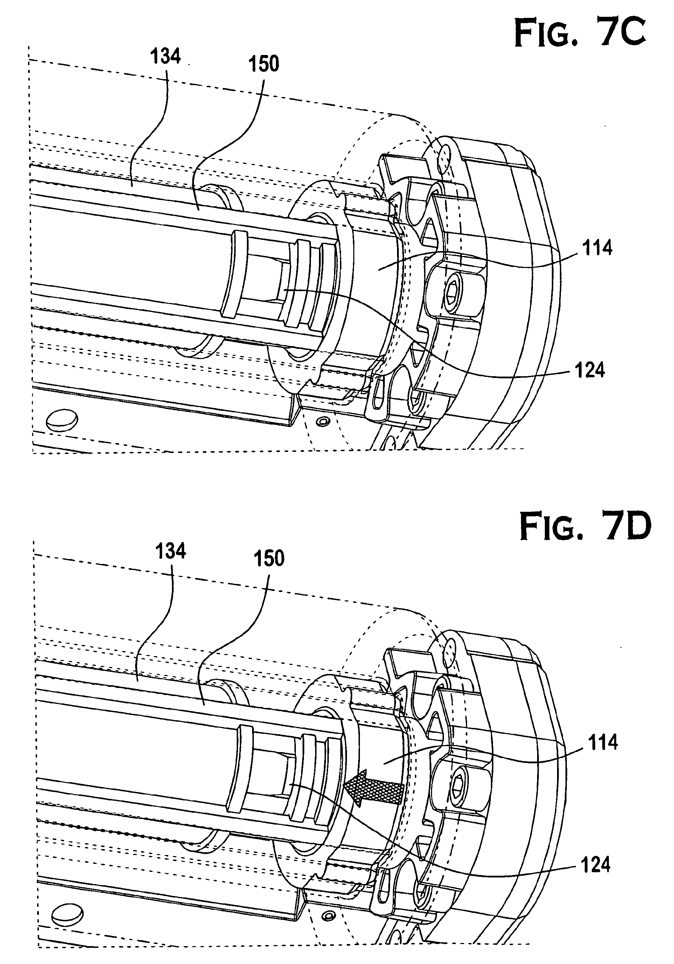

As illustrated in FIG. 6A, the cartridge 104 is inserted into the chamber 116 of the drug infusion pump 102 such that the at least one tab 136 on the outer surface 138 of the barrel 132 of the cartridge 104 engages with and slides within the at least one slot 120. At this point, the cartridge 104 is fully inserted into the drug infusion pump 102 and the piston 114 is fully retracted (see FIG. 7A). The cartridge 104 is then rotated (e.g., clockwise), (see FIGS. 6B, and 7B). Next, the spring retention feature 142 engages with the ledge 143 of the chamber 116 within the drug infusion pump 102 and is increasingly compressed, e.g., from about 10 percent to about 25 percent compressed (see FIGS. 8A - 8C). Near the completion of the cartridge 104 rotation, the at least one arm 150 of the plunger 134 will flex (not shown) so that it can rest on the upward slope of the at least one projection 124 on the outer surface 126 of the piston 114 (see FIG. 7C).

As illustrated in FIG. 7D, the drug infusion pump 102 priming sequence is then initiated such that the piston 114 moves toward the plunger 134. As the piston 114 moves toward the plunger 134, the at least one projection 124 is passively engaged with the opening 152 in the at least one arm 150 of the plunger 134. The priming sequence is halted when a force sensor detects an increase in force due to contact of the distal end 123 of the piston 114 with an inner surface near the first end 144 of the plunger 134 (not shown). At this stage of the priming process, the at least one projection 124 on the piston 114 is seated within the opening 152 in the at least one arm 150, locking the cartridge 104 to the plunger 134 so that the drug cannot be inadvertently delivered during changes in atmospheric pressure or head height.

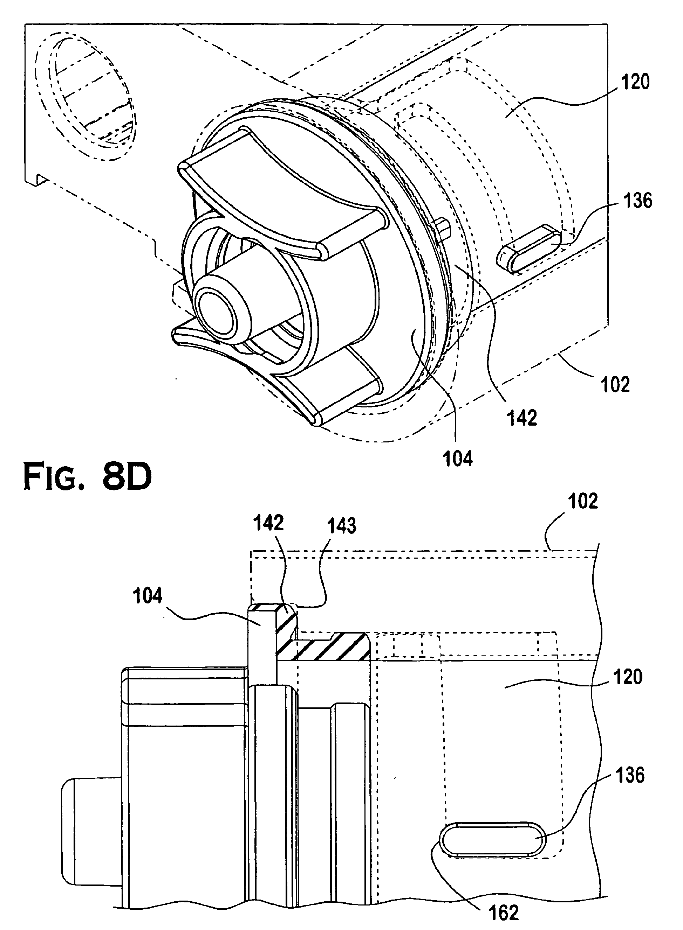

As illustrated in FIG. 8D, simultaneous with the cartridge 104 being locked onto the piston 114, the at least one tab 136 on the barrel 132 moves longitudinally into a recess 162 within the at least one slot 120 in the chamber 116, releasing compression of the spring retention feature 142.

In one exemplary embodiment in which the there are two tabs on the outer surface 138 of the barrel 132 separated by 180 degrees, the cartridge 104 is rotated 90 degrees to lock the cartridge 104 within the chamber 116. In another exemplary embodiment in which there is one tab on the barrel 132 and one slot 120 within the chamber 116, the cartridge 104 is rotated 180 degrees to lock the cartridge 104 within the chamber 116. In other exemplary embodiments in which there are three tabs on the barrel 132 and three slots within the chamber 116, the cartridge 104 is rotated 60 degrees to lock it within the chamber 116. In yet another exemplary embodiment in which there are four tabs on the barrel 132 and four slots within the chamber 116, the cartridge 104 is rotated 45 degrees to lock it within the chamber 116.

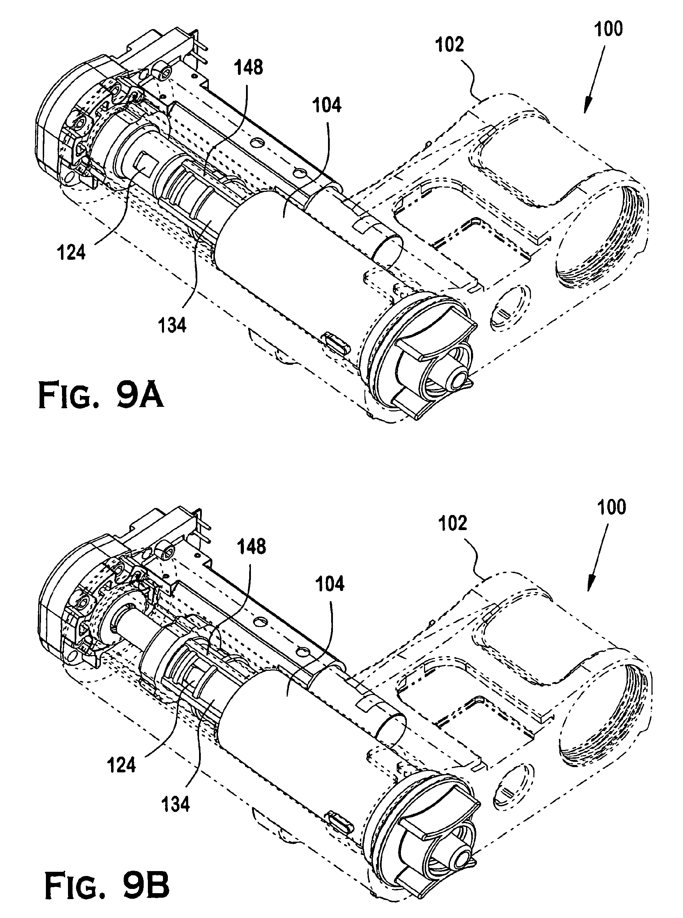

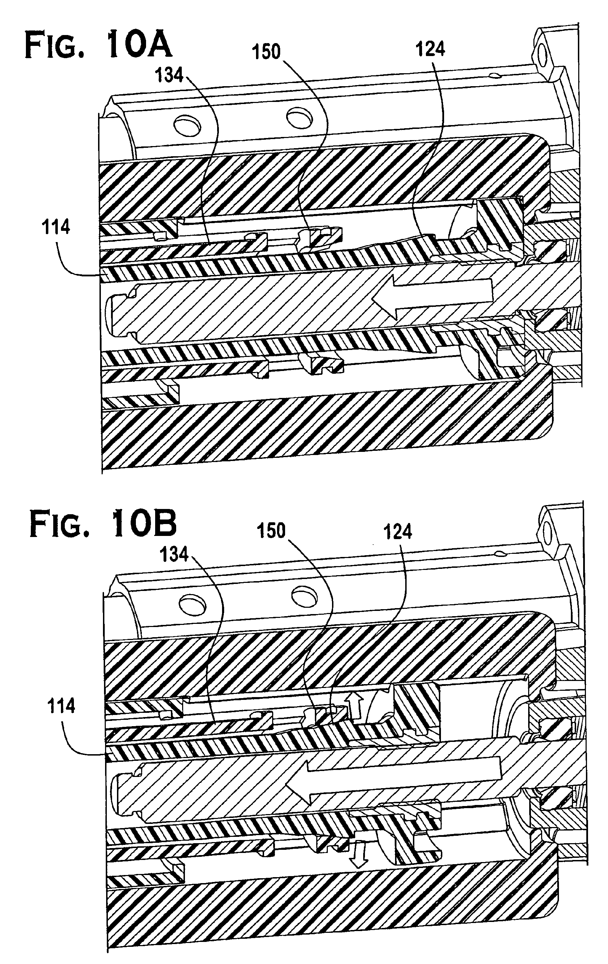

Referring to FIGS. 9A - 9B and 10A - 10C, a sequence of steps in a process for priming a drug infusion pump 102 is illustrated in which the cartridge 104 is partially filled with a drug (e.g., insulin). The provision of an exemplary drug delivery system 100 that may be used with the subject method is depicted in FIGS. 1A and 1B in which like elements of the earlier figures are identified with like numerals.

In the first step of the subject method, a drug delivery system 100 is provided that includes a drug infusion pump 102 and a cartridge 104. The cartridge 104 includes a plunger 134 with an interlock 148 on a distal end 130 according to exemplary embodiments of the present invention (see FIG. 9A).

The cartridge 104 is inserted into the chamber 116 of the drug infusion pump 102 such that the at least one tab 136 one the outer surface 138 of the barrel 132 of the cartridge 104 engages with and slides within the at least one slot 120 (not shown). The cartridge 104 is then rotated (e.g., clockwise; not shown). Next, the spring retention feature 142 engages with the ledge 143 within the chamber 116 of the drug infusion pump 102 and is increasingly compressed, e.g., from about 10 percent to about 25 percent compressed (not shown).

As illustrated in FIGS. 9A and 10A, the drug infusion pump 102 priming sequence is then initiated such that the piston 114 moves toward the plunger 134. As the piston 114 moves toward the plunger 134, the at least one arm 150 of the plunger 134 will flex (see FIG. 10B) so that it can rest on the upward slope of the at least one projection 124 on the outer surface 126 of the piston 114. As illustrated in FIGS. 9B and 10C, the at least one projection 124 is then passively engaged with the opening 152 in the at least one arm 150 of the plunger 134. As illustrated in FIG. 10C, the priming sequence is halted when a force sensor detects an increase in force due to contact of the distal end 123 of the piston 114 with an inner surface near the first end 144 of the plunger 134. At this stage of the priming process, the at least one projection 124 on the piston 114 is seated within the opening 152 in the at least one arm 150, locking the cartridge 104 to the plunger 134 so that the drug cannot be inadvertently delivered during changes in atmospheric pressure or head height. Thus, the locking of the cartridge to the pump can occur prior to, and/or independently of the plunger interlock.

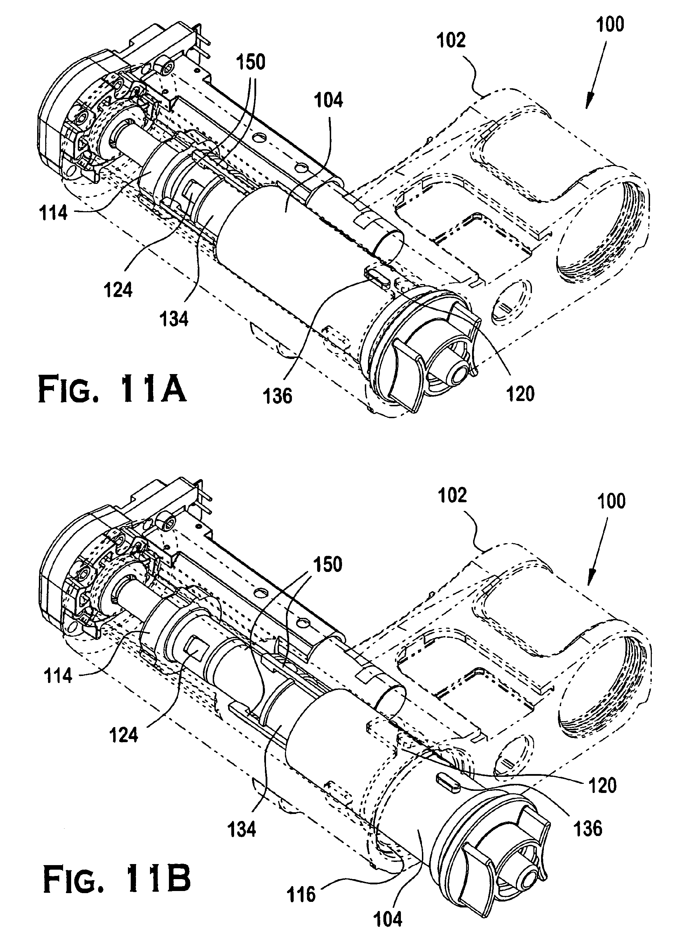

Referring to FIGS. 11A - 11B, a sequence of steps in a process for removing a cartridge 104 from a drug infusion pump 102 is illustrated. The provision of an exemplary drug delivery system 100 that may be used with the subject method is depicted in FIGS. 1A and 1B in which like elements of the earlier figures are identified with like numerals.

As illustrated in FIG. 11A, to remove the cartridge 104 from the chamber 116 of the drug infusion pump 102, the cartridge 104 is rotated in a direction opposite to the direction of rotation during insertion of the cartridge 104 such that the at least one tab 136 on the plunger 134 is disengaged with the at least one slot 120 within the chamber 116 and the at least one projection 124 on the piston 114 is disengaged with the at least one arm 150 on the plunger 134. For example, if a clockwise rotation was used to lock the cartridge 104 within the drug infusion pump 102, then a counter-clockwise rotation would be used to disengage the at least one tab 136 from the at least one slot 120 and the at least one projection 124 from the at least one arm 150. The cartridge 104 would then simply be pulled out of the chamber 116 until completely removed, as shown in FIG. 11B.

It will be recognized that equivalent structures may be substituted for the structures illustrated and described herein and that the described embodiment of the invention is not the only structure, which may be employed to implement the claimed invention. In addition, it should be understood that every structure described above has a function and such structure can be referred to as a means for performing that function. While embodiments of the present invention have been shown and described herein, it will be obvious to those skilled in the art that such embodiments are provided by way of example only. Numerous variations, changes, and substitutions will now occur to those skilled in the art without departing from the invention.

It should be understood that various alternatives to the embodiments of the invention described herein may be employed in practicing the invention. It is intended that the following claims define the scope of the invention and that methods and structures within the scope of these claims and their equivalents be covered thereby.

## Claims

1. A method of priming a drug infusion pump (102), comprising:
providing the drug infusion pump having:
a chamber (116) for retaining a cartridge (104), the chamber having at least one slot (120) on an inner surface (121);
a piston (114) having at least one projection (124) on an outer surface (126); and
a motor for moving the piston to deliver a drug from the cartridge; and
providing a cartridge having:
a hollow barrel (132) configured to receive and store a drug, the barrel having at least one tab (136) on an outer surface (138); and
a plunger (134) slidably inserted into the barrel and movable within the barrel, the plunger having an interlock (148) on a first end (146);
inserting the cartridge into the chamber of the drug infusion pump;
rotating the cartridge to engage the at least one tab on the barrel with the at least one slot in the chamber to secure the cartridge within the drug infusion pump and to align the interlock with the at least one projection on the piston;
moving the piston toward the cartridge;
sensing a resistive force to indicate that the piston is in contact the plunger; and
engaging the interlock of the plunger with the at least one projection on the piston;
**characterised in that** the drug delivery system further comprises a cartridge anti-rotation mechanism wherein the cartridge anti-rotation mechanism comprises at least one rail (154) on an outer surface of the plunger that mates with and slides within a groove (158) on an inner surface of the barrel.

2. The method of claim 1, wherein the provided cartridge is partially filled with the drug.

3. The method of claim 2, wherein the drug is insulin.

4. The method of claim 1, wherein the interlock comprises at least one flexible arm (150) and an opening (152) for receiving the at least one projection on the piston.

5. The method of claim 1, wherein the resistive force sensed is greater than a force required to engage the interlock and is less than a force required to advance the plunger.

6. A cartridge (104) for use in a drug infusion pump (102), the cartridge comprising:
a hollow barrel (132) configured to receive and store a drug, the barrel having at least one tab (136) on an outer surface (138) that engages with at least one slot (120) in a chamber (116) of the drug infusion pump; and
a plunger (134) slidably inserted into the barrel and movable within the barrel, the plunger having an interlock (148) on a first end (146) for engaging a piston (114) within the drug infusion pump;
**characterised in that** the cartridge further comprises a cartridge anti-rotation mechanism wherein the cartridge anti-rotation mechanism comprises at least one rail (154) on an outer surface of the plunger that mates with and slides within a groove (158) on an inner surface of the barrel.

7. The cartridge of claim 6, wherein the interlock comprises at least one flexible arm (150) on the plunger and an opening (152) for receiving the at least one projection on the piston.

8. The cartridge of claim 6, wherein the cartridge further comprises a gasket (142) that provides a spring force to retain the cartridge within the drug infusion pump.

9. A drug delivery system (100) comprising:
a drug infusion pump(102) having:
a chamber (116) for retaining a cartridge (104), the chamber having at least one slot (120) on an inner surface (121);
a piston (114) having at least one projection (124) on an outer surface (126); and a motor for moving the piston to deliver a drug from the cartridge; and
a cartridge according to any one of claims 6 to 8.

## Patentansprüche

1. Verfahren zum Priming einer Arzneimittelinfusionspumpe (102), das Folgendes aufweist:
Bereitstellen der Arzneimittelinfusionspumpe mit:
einer Kammer (116) zum Aufnehmen einer Kartusche (104), wobei die Kammer mindestens einen Schlitz (120) an einer Innenseite (121) aufweist;
einem Kolben (114), der mindestens einen Vorsprung (124) an einer Außenseite (126) aufweist; und
einem Motor zum Bewegen des Kolbens für die Lieferung eines Arzneimittels aus der Kartusche; und
Bereitstellen einer Kartusche mit:
einem hohlen Zylinder (132), der zum Aufnehmen und Lagern eines Arzneimittels ausgebildet ist, wobei der Zylinder mindestens eine Nase (136) auf einer Außenseite (138) aufweist; und
einem Stempel (134), der gleitend in den Zylinder eingeführt wird und innerhalb des Zylinders verschiebbar ist, wobei der Stempel eine Verriegelung (148) an einem ersten Ende (146) aufweist;
Einsetzen der Kartusche in die Kammer der Arzneimittelinfusionspumpe;
Drehen der Kartusche zum Eingreifen der mindestens einen Nase auf dem Zylinder in den mindestens einen Schlitz in der Kammer, um die Kartusche innerhalb der Arzneimittelinfusionspumpe zu sichern und die Verriegelung mit dem mindestens einen Vorsprung an dem Kolben auszurichten;
Bewegen des Kolbens in Richtung der Kartusche;
Erfassen einer Widerstandskraft, um anzuzeigen, dass der Kolben mit dem Stempel in Kontakt ist; und
Ineingrifftreten der Verriegelung von dem Stempel mit dem mindestens einen Vorsprung an dem Kolben;
**dadurch gekennzeichnet, dass** das Arzneimittelabgabesystem ferner einen Kartuschen-Antirotationsmechanismus aufweist, wobei der Kartuschen-Antirotationsmechanismus mindestens eine Schiene (154) an einer Außenseite des Stempels aufweist, die mit einer Vertiefung (158) auf einer Innenseite des Zylinders zusammenpasst und darin gleitet.

2. Verfahren nach Anspruch 1, wobei die bereitgestellte Kartusche teilweise mit dem Arzneimittel gefüllt ist.

3. Verfahren nach Anspruch 2, wobei das Arzneimittel Insulin ist.

4. Verfahren nach Anspruch 1, wobei die Verriegelung mindestens einen flexiblen Arm (150) und eine Öffnung (152) zum Aufnehmen des mindestens einen Vorsprungs an dem Kolben aufweist.

5. Verfahren nach Anspruch 1, wobei die erfasste Widerstandskraft größer als eine Kraft ist, die für den Eingriff der Verriegelung benötigt wird, und niedriger ist als eine Kraft, die für den Vorschub des Stempels benötigt wird.

6. Kartusche (104) zur Verwendung in einer Arzneimittelinfusionspumpe (102), wobei die Kartusche Folgendes aufweist:
einen hohlen Zylinder (132), der zum Aufnehmen und
Lagern eines Arzneimittels ausgebildet ist, wobei der Zylinder mindestens eine Nase (136) an einer Außenseite (138) aufweist, die mit mindestens einem Schlitz (120) in einer Kammer (116) der Arzneimittelinfusionspumpe in Eingriff tritt; und
einen Stempel (134), der gleitend in den Zylinder eingeführt wird und innerhalb des Zylinders verschiebbar ist, wobei der Stempel eine Verriegelung (148) an einem ersten Ende (146) für den Eingriff mit einem Kolben (114) innerhalb der Arzneimittelinfusionspumpe aufweist;
**dadurch gekennzeichnet, dass** die Kartusche ferner einen Kartuschen-Antirotationsmechanismus aufweist, wobei der Kartuschen-Antirotationsmechanismus mindestens eine Schiene (154) an einer Außenseite des Stempels aufweist, die mit einer Vertiefung (158) auf einer Innenseite des Zylinders zusammenpasst und darin gleitet.

7. Kartusche nach Anspruch 6, wobei die Verriegelung mindestens einen flexiblen Arm (150) an dem Stempel und eine Öffnung (152) zum Aufnehmen des mindestens einen Vorsprungs an dem Kolben aufweist.

8. Kartusche nach Anspruch 6, wobei die Kartusche ferner eine Dichtung (142) aufweist, die eine Federspannung bereitstellt, um die Kartusche innerhalb der Arzneimittelinfusionspumpe festzuhalten.

9. Arzneimittelabgabesystem (100), das Folgendes aufweist:
eine Arzneimittelinfusionspumpe (102) mit:
einer Kammer (116) zum Aufnehmen einer Kartusche (104), wobei die Kammer mindestens einen Schlitz (120) an einer Innenseite (121) aufweist;
einem Kolben (114), der mindestens einen Vorsprung (124) an einer Außenseite (126) aufweist; und
einem Motor zum Bewegen des Kolbens für die Lieferung eines Arzneimittels aus der Kartusche; und
eine Kartusche nach einem der Ansprüche 6 bis 8.

## Revendications

1. Procédé pour amorcer une pompe à perfusion de médicament (102), comprenant les opérations consistant à :
procurer une pompe à perfusion de médicament comportant :
une chambre (116) pour retenir une cartouche (104), la chambre ayant au moins une fente (120) sur sa surface intérieure (121) ;
un piston (114) ayant au moins un bossage (124) sur sa surface extérieure (126) ; et
un moteur électrique pour déplacer le piston afin d'administrer un médicament provenant de la cartouche ; et
procurer une cartouche comportant :
un cylindre creux (132) configuré pour recevoir et stocker un médicament, le cylindre ayant au moins une languette (136) sur sa surface extérieure (138) ; et
un piston (134) inséré coulissant dans le cylindre et mobile à l'intérieur du cylindre, le piston ayant un verrouillage (148) sur sa première extrémité (146) ;
insérer la cartouche dans la chambre de la pompe à perfusion de médicament ;
faire tourner la cartouche pour mettre en prise ladite languette sur le cylindre avec ladite fente dans la chambre afin de bien fixer la cartouche à l'intérieur de la chambre de la pompe à perfusion de médicament et d'aligner le verrouillage avec ledit bossage sur le piston ;
déplacer le piston vers la cartouche ;
sentir une force de résistance qui indique que le piston est en contact avec le piston ; et
solidariser le verrouillage du piston avec ledit bossage sur le piston ;
**caractérisé en ce que** le système d'administration de médicament comprend en outre un mécanisme antirotation de cartouche qui comprend au moins un rail (154) sur la surface extérieure du piston qui s'accouple avec une rainure (158) sur la surface intérieure du cylindre et coulisse à l'intérieur de celle-ci.

2. Procédé selon la revendication 1, dans lequel la cartouche fournie est partiellement remplie du médicament.

3. Procédé selon la revendication 2, dans lequel le médicament est de l'insuline.

4. Procédé selon la revendication 1, dans lequel le verrouillage comprend au moins un bras flexible (150) et une ouverture (152) pour recevoir ledit bossage sur le piston.

5. Procédé selon la revendication 1, dans lequel la force de résistance ressentie est supérieure à la force nécessaire pour enclencher le verrouillage et inférieure à la force nécessaire pour faire avancer le piston.

6. Cartouche (104) à employer dans une pompe à perfusion de médicament (102), la cartouche comprenant :
un cylindre creux (132) configuré pour recevoir et stocker un médicament, le cylindre ayant au moins une languette (136) sur sa surface extérieure (138) qui s'engage dans au moins une fente (120) dans une chambre (116) de la pompe à perfusion de médicament ; et
un piston (134) inséré coulissant dans le cylindre et mobile à l'intérieur du cylindre, le piston ayant un verrouillage (148) sur sa première extrémité (146) pour s'enclencher avec un piston (114) à l'intérieur de la pompe à perfusion de médicament ;
**caractérisée en ce que** la cartouche comprend en outre un mécanisme antirotation de cartouche qui comprend au moins un rail (154) sur la surface extérieure du piston qui s'accouple avec une rainure (158) sur la surface intérieure du cylindre et coulisse à l'intérieur de celle-ci.

7. Cartouche selon la revendication 6, dans laquelle le verrouillage comprend au moins un bras flexible (150) sur le piston et une ouverture (152) pour recevoir ledit bossage sur le piston.

8. Cartouche selon la revendication 6, dans laquelle la cartouche comprend en outre un joint (142) qui assure une force élastique pour retenir la cartouche à l'intérieur de la pompe à perfusion de médicament.

9. Système (100) d'administration de médicament comprenant :
une pompe à perfusion de médicament (102) comportant :
une chambre (116) pour retenir une cartouche (104), la chambre ayant au moins une fente (120) sur sa surface intérieure (121) ;
un piston (114) ayant au moins un bossage (124) sur sa surface extérieure (126) ; et
un moteur électrique pour déplacer le piston afin d'administrer un médicament provenant de la cartouche ; et
une cartouche selon l'une quelconque des revendications 6 à 8.
